# EUROPEAN PATENT SPECIFICATION

(11) **EP 1 109 761 B1**
(45) Date of publication and mention of the grant of the patent: **07.05.2003**
(21) Application number: 99942143.1
(22) Date of filing: 12.08.1999
(51) Int. Cl.: C07C 15/08, C07C 1/00, C07C 5/22

(54) **PARA-XYLENE PRODUCTION PROCESS**
HERSTELLUNGSVERFAHREN FÜR PARA-XYLEN
PROCEDE DE PRODUCTION DE PARA-XYLENE

(30) Priority: 25.08.1998 US 139463; 25.08.1998 US 139465
(43) Date of publication of application: 27.06.2001
(73) Proprietor: EXXONMOBIL OIL CORPORATION, Fairfax, VA 22037 (US)
(72) Inventor: ABICHANDANI, Jeevan, Sahib, Voorhees, NJ 08043 (US); BECK, Jeffrey, Scott, Burlington, NJ 08016 (US); BROWN, Stephen, Harold, Princeton, NJ 08540 (US); CIMINI, Ronald, Joseph, Sewell, NJ 08080 (US); KWOK, Selma, Voorhees, NJ 08043 (US); JOHNSON, Ivy, Dawn, Lawrenceville, NJ 08648 (US); LIGURAS, Dimitris, Konstantine, Mantua, NJ 08051 (US); STERN, David, Lawrence, Mount Laurel, NJ 08054 (US)
(74) Representative: Dew, Melvyn John
(86) International application number: US9918353
(87) International publication number: WO00010944

(56) References cited:
- US-A- 3 948 758

## Description

This invention relates to a process for producing para-xylene from a C₈+ aromatic feedstock.

*Para-* and *ortho*-xylenes are valuable chemical intermediates. In a petrochemical complex, they are produced in large part by recovery of these compounds from both the crude distillation and the C₈ heart cut of the reformer. Recovery is accomplished via several selective separation processes, such as C₈ heart-cut distillation to yield high-purity *ortho*-xylene ("*ortho*-splitter"), and selective crystallization or sorption processes. The remainder from these separation technologies involves mostly *meta-* and *ortho*-xylenes, ethylbenzene, and benzene, with relatively low concentrations of other aromatics.

Several technologies exist for upgrading the chemical value of this recycle stream. For example, there are commercial processes for converting this stream into one containing an equilibrium mixture of xylenes―that is, roughly 50 weight percent (wt.%) *meta*-xylene, and 25 wt.% each of *para-* and *ortho*-xylenes―via isomerization. These processes also reduce the ethylbenzene concentration in this recycle loop through mechanisms such as cracking, or hydrocracking (hydrogenation of the ethylene thus formed from ethylbenzene cracking by means of a functional metal).

Although these processes have been practiced commercially for many years, significant improvements could be realized if it were possible to significantly reduce the capital costs of the isomerization complex. The commercial processes that are presently being used require large capacity processing vessels to produce a relatively small amount of product. Therefore, it is desirable to find a method that increases the amount of product that can be produced by existing equipment and also allow new plants using smaller capacity equipment to produce the equivalent amount of product.

Principal sources of C₈ aromatics mixtures are catalytically reformed naphthas and pyrolysis distillates. The C₈ aromatic fractions from these sources vary quite widely in composition but will usually be in the range of 10 wt.% to 32 wt.% ethylbenzene (EB) with the balance being 50 wt.% *meta*-xylene and 25 wt.% each of *para*-xylene and *ortho*-xylene.

Individual isomer products may be separated from the naturally occurring mixtures by appropriate physical methods. Ethylbenzene may be separated by fractional distillation, although this is a costly operation. *Ortho*-xylene may be separated by fractional distillation, and it is so produced commercially. *Para*-xylene may be separated from the mixed isomers by fractional crystallization, selective adsorption, or membrane separation.

The boiling point of ethylbenzene is very close to those of *para*-xylene and *meta*-xylene. As a result, complete removal of ethylbenzene from the C₈⁺ aromatics mixture by conventional methods, e.g., distillation, is usually impractical. An ethylbenzene separation column may be used in an isomenzer-separator loop or the ethylbenzene may be converted catalytically in the isomerizer-separator loop.

In many processes for xylene isomerization, the conversion of ethylbenzene is not maximized because of the need to control the competing reactions which convert xylenes to less valuable compounds. Thus, when ethylbenzene is catalytically converted, the primary consideration for selecting the operating conditions is to minimize xylene losses from transalkylation of xylenes.

US-A-3948758 teaches the manufacture of a xylene isomer by supplying a feedstream containing C8 hydrocarbons to a process loop which separates the desired isomer, subjects remaining hydrocarbons to isomerisation, fractionates the resulting effluent and returns a part of the effluent to the separation step; wherein the effluent is fractionated together with the hydrocarbon products obtained by reacting a C8 aromatic fraction with hydrogen in contact with a zeolite under conversion conditions, to provide recycle and fresh feed for the separation step.

The present invention seeks to provide a process in which the para-xylene can be produced from a C₈⁺ aromatic feedstock with improved efficiency and at lower cost. Accordingly, the invention resides in a process for producing *para*-xylene from a C₈⁺ aromatic feedstock which contains xylenes and ethylbenzene, the process comprising the steps of:
(a) subjecting said feedstock to ethylbenzene conversion in a first stage to provide a first effluent stream;
(b) removing C₉⁺ aromatics and, optionally, *ortho*-xylene, from said first effluent stream to provide a first depleted stream;
(c) recovering *para*-xylene from said first depleted stream to provide a second depleted stream;
(d) isomerizing xylenes in said second depleted stream to provide a second effluent stream;
(e) recycling said second effluent stream into said first depleted stream prior to *para*-xylene recovery; and
(f) recycling a step stream from said second depleted stream into said feedstock.

Preferably, the first stage (a) also effects isomerization of the xylenes in the feedstock. The xylene isomerization can be accomplished in a dual-bed reactor, wherein the ethylbenzene conversion takes place in the first section of the reactor and the xylene isomerization takes place in the second section of the reactor, or in a separate reactor that is preferably located immediately downstream of the ethylbenzene conversion reactor.

The dual stage process of the invention has the advantage of isomerizing xylenes more efficiently than a conventional single stage process. The first ethylbenzene conversion/isomerization stage converts the ethylbenzene in the feedstock to benzene and ethane. The effluent is processed to remove C₉⁺ aromatics, and optionally *ortho*-xylene, to provide a first depleted stream and then processed to remove benzene, toluene and lighter hydrocarbons to provide a second depleted stream. Thus, when the second depleted stream is processed in the second isomerization stage, the volume of the effluent from the first ethylbenzene conversion/isomerization stage is reduced and the effluent is rich in the xylenes that are targeted by the process. Accordingly, the vessels in the second isomerization stage can be smaller and operate more efficiently.

The recycle loop in the second isomerization stage, which recycles the second effluent stream into the first depleted stream, also provides the advantage of maximizing the production of *para*-xylene. Since *ortho-xylene* and *meta*-xylene are not removed from the process during the second isomerization stage, the xylene rich effluent stream is recycled until all of the xylenes are either converted to *para*-xylene, or are removed from the process as lower order hydrocarbons in the benzene/toluene separator.

Preferably, toluene is added to the feedstock prior to the first stage to minimize the loss of xylene during the ethylbenzene conversion stage of the process. The toluene can be added from a source external to the process, or by co-feeding toluene recovered from the first effluent stream. In some cases, toluene can be obtained from a combination of an external source and toluene recovered from the first effluent stream. Conveniently, the toluene is removed from the first depleted stream prior to the *para*-xylene recovery. After the toluene has been added, the preferred toluene concentration in the feedstock is between 1% and 25% by weight, more preferably from 10 to 20% by weight. Preferably, the toluene is added so as to increase the ratio of toluene to xylenes in the feedstock to a range of from I to 10 to 1 to 2, most preferably from 1 to 4 to 1 to 6.

It has been found that co-fed toluene reduces xylene-losses in ethylbenzene conversion reactions (i.e., in the bed of an ethylbenzene conversion reactor or in the top bed of a dual ethylbenzene conversion/xylene isomerization reactor), from 1.7 wt.% at 90% ethylbenzene conversion without co-fed toluene, to 0.8% with 19% co-fed toluene. In the xylene isomerization unit, co-fed toluene may suppress side reactions such as xylene disproportionation since toluene is one of the products of this reaction that is converted.

In accordance with the invention, a slip stream is taken from the second depleted stream after *para*-xylene is recovered and prior to the isomerization stage, and recycled into the feedstock. In a preferred embodiment, a further slip stream is taken from the second depleted stream after *para*-xylene is recovered and prior to the isomerization stage, and recycled into the first effluent stream after ethylbenzene conversion.

Recycling part of the second depleted stream provides a means for removing any C₉⁺ aromatics and *ortho*-xylene from the second depleted stream and also provides a means for controlling the recycle flow of the second effluent stream. Up to 50%, and more preferably from 5% to 25%, by weight of the second depleted stream can be recycled through the slip stream(s).

### Feedstock

In general, any aromatic C₈ mixture containing ethylbenzene and xylenes may be used as feedstock to the process of this invention. Generally, such a feedstock mixture will typically have a C₉⁺ aromatics content of from 0 wt.% to 30 wt.%, an ethylbenzene content of 5 wt.% to 50 wt.%; an *ortho-*xylene content of 0 wt.% to 35 wt.%, a *meta*-xylene content of 20 wt.% to 90 wt.%, and a *para*-xylene content of 0 wt.% to 25 wt.%. For example, the feedstock may contain 10 wt.% to 15 wt.% ethylbenzene with the balance made up of xylenes and C₉⁺ aromatics. Preferably, the feedstock will contain at least 30 wt.%, more preferably at least 50 wt.%, *meta*-xylene. Feedstocks meeting this standard are termed "*meta*-xylene-rich" feedstocks. Moreover, the feedstock in addition to the above C₈ aromatic mixture may contain non-aromatic hydrocarbons, i.e., naphthenes and paraffins in an amount up to 30 wt.%.

### Ethylbenzene Conversion Stage

In accordance with the present invention, the above described feedstock is initially contacted with a catalyst system under suitable conversion conditions to effect ethylbenzene conversion, while minimizing xylene losses. Conditions effective to accomplish ethylbenzene conversion include a temperature of 200°C to 550°C, a pressure of from 0 MPag to 6.89 MPag (0 psig to 1,000 psig), a WHSV of between 0.1 hr¹ and 200 hr and an H₂/HC molar ratio of between 0.2 and 10. More preferably, the conversion conditions include a temperature of 325°C to 475°C, a pressure of 345 kPag to 2.76 MPag (50 psig to 400 psig), a WHSV of between 3 hr¹ and 50 hr¹, and an H₂/HC molar ratio of between 1 and 5.

The ethylbenzene conversion can proceed by dealkylation to produce benzene and ethane and/or ethylene or by way of isomerization to produce additional xylenes.

Examples of catalysts suitable for use in the ethylbenzene conversion stage of the invention include intermediate pore size zeolites such as ZSM-5 (U.S. Pat. Nos. 3,702,886 and Re. 29,948); ZSM-11 (U.S. Pat. No. 3,709,979); ZSM-12 (U.S. Pat. No. 3,832,449); ZSM-22 (U.S. Pat. No. 4,556,477); ZSM-23 (U.S. Pat. No. 4,076,842); ZSM-35 (U.S. Pat. No. 4,016,245); ZSM-48 (U.S. Pat. No. 4,397,827); ZSM-57 (U.S. Pat. No. 4,046,685); ZSM-58 (U.S. Pat. No. 4,417,780); and SAPO-5, SAPO-11 and SAPO-41 (U.S. Pat. No. 4,440,871).

The ethylbenzene conversion catalyst is preferably modified so as to enhance its activity and/or selectivity for the desired ethylbenzene conversion reaction. Suitable modification methods include silica selectivation, coke selectivation, steaming, adding a hydrogenation/dehydrogenation functional metal, and combinations thereof, as well as other techniques that are known in the art. For example, the modified catalyst can comprise a silica-bound intermediate pore size molecular sieve, e.g., ZSM-5, which has been selectivated by being coated with at least one coating of an organosilicon selectivating agent in a liquid carrier and subsequently calcining the catalyst in an oxygen-containing atmosphere. Coke selectivation can be performed by contacting the catalyst with a decomposable organic compound under conditions sufficient to decompose the organic compound while not damaging the catalyst. Steaming can also be performed to modify the activity of the catalyst as is known in the art. By virtue of such modification, the selectivity of the catalyst for the desired ethylbenzene conversion reaction can be increased.

The ethylbenzene conversion stage of the process of the invention may be operated at extremely high ethylbenzene conversion levels, typically greater than 50%, e.g., greater than 70%, e.g., greater than 80%, e.g., greater than 85%, e.g., 90% or more by weight.

Preferably, the ethylbenzene conversion stage of the process of the invention also effects some isomerization of the xylenes in the feedstock. This can be accomplished using a dual-bed reactor, in which the ethylbenzene conversion takes place in the first section of the reactor and the xylene isomerization takes place in the second section of the reactor, or in a separate reactor that is preferably located immediately downstream of the ethylbenzene conversion reactor. The catalyst used in the xylene isomerization section or reactor is preferably an intermediate pore size zeolite, such as ZSM-5, which has been modified in known manner so as to enhance its activity and/or selectivity for the desired xylene isomerization reaction.

Preferably, the ethylbenzene conversion catalyst and xylene isomerization catalyst contain a hydrogenation metal, such as Pt, Pd, Ni, Rd, Fe, W and Re.

Suitable combined ethylbenzene conversion/xylene isomerization reaction stages are disclosed in U.S. Patent Nos. 4,899,011 and 5,689,027.

### Xylene Isomerization Loop

The effluent from the ethylbenzene conversion stage is fed to a xylene splitter to remove C₉⁺ aromatics and, optionally, *ortho*-xylene before being passed to a conventional xylene isomerization loop. In this loop, *para*-xylene is initally recovered as a product stream, typically by selective sorption or crystallization, and then the *para*-depleted effluent is fed to a further xylene isomerization reactor, where the xylenes are converted back to thermal equilibrium concentration. The effluent from the xylene isomerization reactor is then recycled back to the *para*-xylene recovery unit.

The further xylene isomerization reactor may use the same or different catalyst as that used in the xylene isomerization stage of the ethylbenzene conversion unit and may be operated under the same or different conditions as said xylene isomerization stage. It may, however, be desirable to operate the further xylene isomerization reactor under liquid phase conditions so as to increase ease of operation and decrease capital costs.

The invention will now be more particularly described with reference to the accompanying drawings, wherein:
Figure 1 is a diagram of an ethylbenzene conversion/xylene isomerization process known in the prior art.
Figure 2 is a diagram of a process according to a first example of the invention having two stages of xylene isomerization.
Figure 3 is a diagram of a process according to a second example of the invention having two stages of xylene isomerization and toluene recycled into the feedstock.

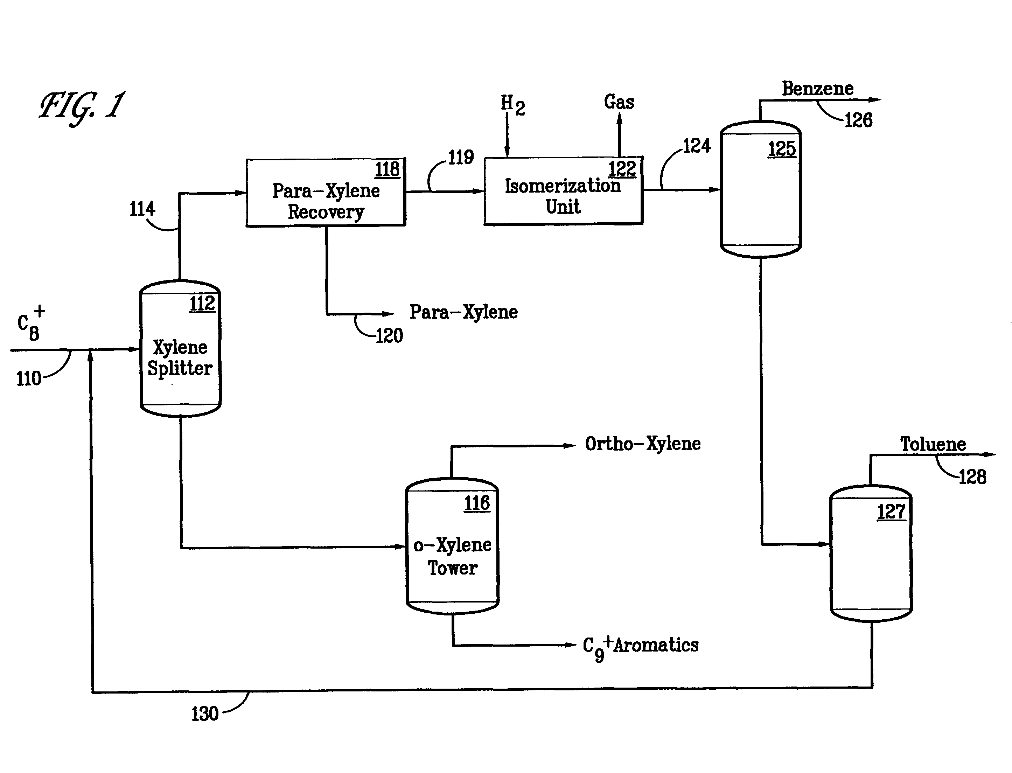

Figure 1 shows a xylene isomerization process known in the prior art wherein a feedstock 110 made up of C₈⁺ aromatics is fed into a xylene splitter 112, which removes C₉⁺ aromatics and *ortho-*xylene from the main feed stream 114. The C₉⁺ aromatics and *ortho-*xylene are separated in an *ortho-*xylene tower 116 and taken off from the process as by-products. The main feed stream 114 is then processed in a *para*-xylene recovery unit 118 where *para*-xylene is removed from the feedstock as a product 120. The residual feedstock is processed in an isomerization unit 122 where xylenes in the feedstock are converted to thermal equilibrium concentration and ethylbenzene may be converted to lower order aromatics. Benzene 126 and toluene 128 are separated from the effluent of the isomerization unit 124 and the depleted effluent 130 is recycled back into the feedstock 110, upstream of the xylene splitter 112.

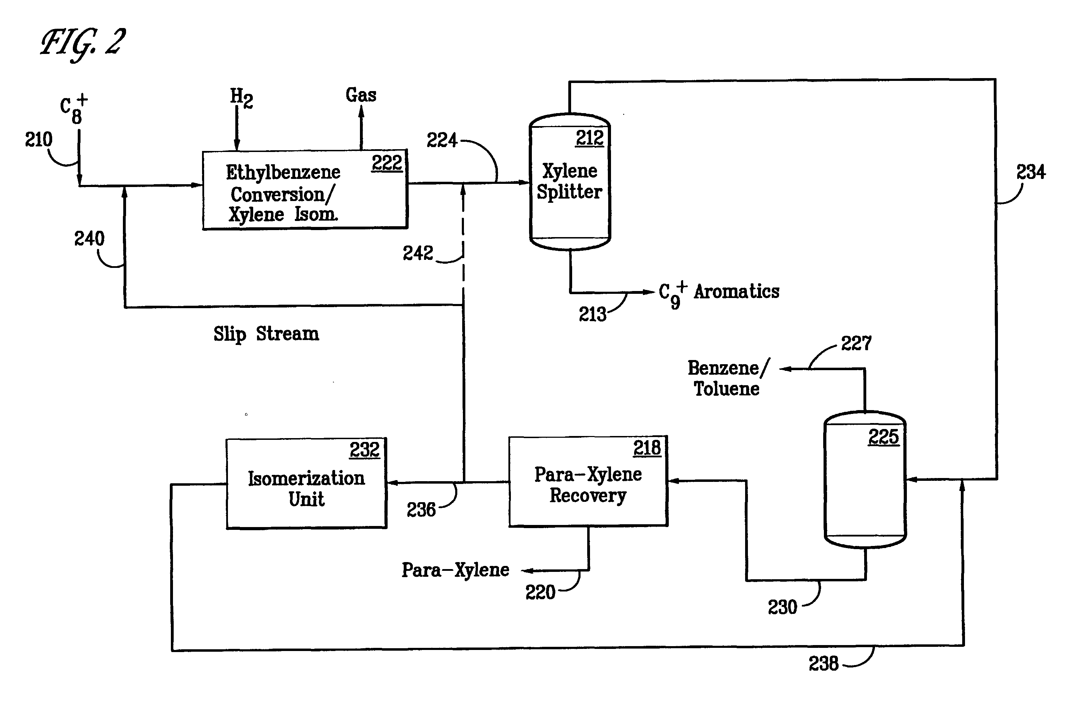

Figure 2 shows a xylene production process according to a first example of the present invention having two isomerization stages. A C₈⁺ aromatics feedstock 210 is fed into an ethylbenzene conversion/xylene isomerization reactor 222 where the ethylbenzene in the feedstock is converted in a first section of the reactor. The feedstock is isomerized in the second section of the reactor and xylenes are converted to thermal equilibrium concentration. The effluent from the ethylbenzene conversion/xylene isomerization reactor 222 provides a first effluent stream 224 that is sent to a xylene splitter 212, which removes C₉⁺ aromatics 213 (and in some embodiments of the invention *ortho-*xylene) as by-products and provides a first depleted stream 234. A separator 225 removes benzene and toluene 227 from the first depleted stream 234 as by-products. The first depleted stream with benzene and toluene removed 230 is processed in a *para*-xylene recovery unit 218 where *para*-xylene is removed as a product 220, preferably by selective sorption and/or crystallization. The effluent from the *para*-xylene recovery unit 218 provides a second depleted stream 236 that is fed to an isomerization unit 232, where xylenes are converted to the thermal equilibrium concentration and most of the ethylbenzene is converted to benzene and ethane. Second effluent stream 238 from the isomerization unit 232 is comprised primarily of xylenes, in the proportion of approximately 50% *meta*-xylene, *25% ortho*-xylene and 25% *para*-xylene. The second effluent stream 238 is recycled into the first depleted stream 234, upstream of the benzene/toluene separator 225. The recycling of the xylene rich second effluent stream 238 from the isomerization unit maximizes the production of *para*-xylene. The size of the *para*-xylene recovery unit 218 and the isomerization unit 232 can be reduced because the volume of the material treated in these units is reduced by removing C₉⁺ aromatics and C₇₋ aromatics upstream in the process.

Part of the effluent 236 from the *para*-xylene recovery unit 218 is recycled as a slip stream 240 into the feedstock 210 upstream of the ethylbenzene conversion/xylene isomerization reactor 222 and optionally as a slip stream 242 into the first effluent stream 224 of the reactor 222. The slip stream 240 provides the advantage of allowing the feedstock 210 to be enriched with C₈ aromatics and also provides a means for controlling the recycling of C₈ aromatics through the *para*-xylene recovery unit 218 and the isomerization unit 232. The slip stream 240 provides a means for removing C₉⁺ aromatics, and optionally *ortho*-xylene, from the isomerization/*para*-xylene recovery unit stage 218/232 of the process.

Typically, the slip streams 240, 242 recycle up to 50% by weight, and preferably 5% to 25% by weight, of the second depleted stream 236. Where both slip streams 240 and 242 are employed, the amount of recycle to the feedstock 210 through the slip stream 240 is typically from 5 to 1 to 1 to 1, and preferably 2 to 1, to the amount of recycle to first effluent stream 224 through the slip stream 242. The amount of flow through the slip streams 240 and 242 is selected based on the composition of the second depleted stream 236, the composition of the feedstock 210 and the catalysts used in the ethylbenzene conversion/xylene isomerization reactor 222 and the isomerization reactor 232.

A conventional xylene isomerization plant, such as that illustrated schematically in Figure 1, can be modified to provide a process as shown in Figure 2 by adding an ethylbenzene conversion unit upstream of the xylene splitter 112 and reconfiguring the process streams. The process streams are reconfigured by connecting the light aromatics outlet 114 from the xylene splitter 112 to the inlet of the benzene separation unit 125 downstream of the isomerization unit 122 and connecting the heavy aromatics outlet 130 of the toluene separation unit 127 to the inlet to the *para*-xylene recovery unit 118. A slip stream is taken off *para*-xylene recovery unit effluent 119 and recycled into the feedstock 110 upstream of the ethylbenzene conversion unit. Such a modification would require minimal capital costs, since the "basic" hardware is already in place. Expected savings for a grass roots design would be even greater.

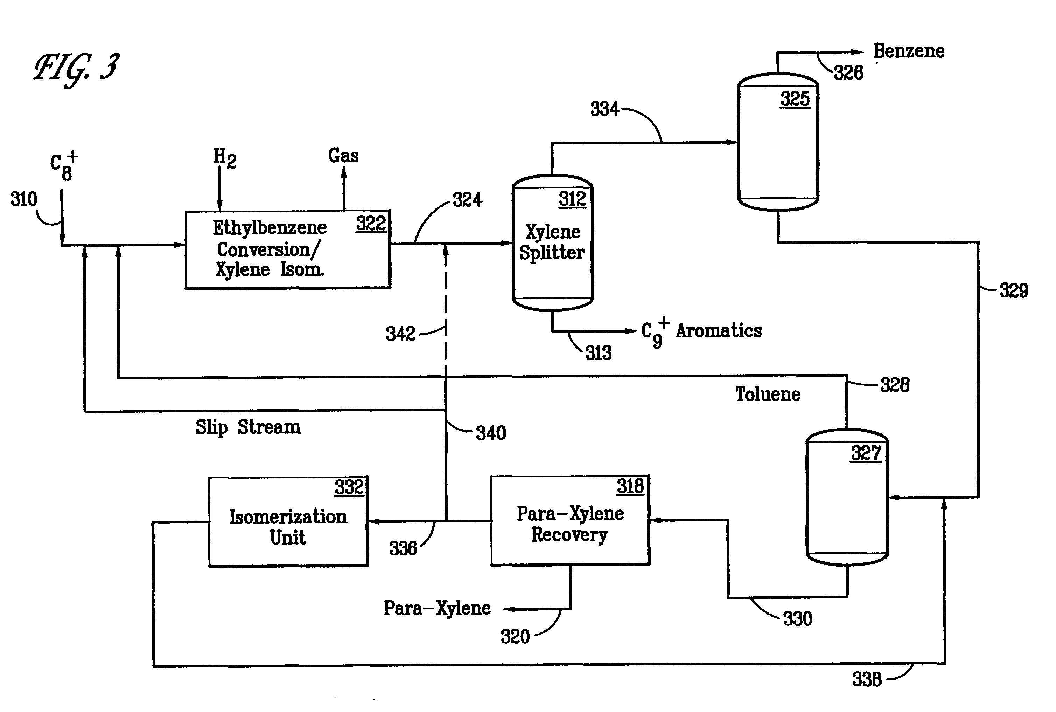

Figure 3 shows a xylene production process according to a second example of the invention which has a second isomerization stage and recycles toluene into the feedstock. A C₈⁺ aromatics feedstock 310 is fed into an ethylbenzene conversion/xylene isomerization reactor 322 where the ethylbenzene in the feedstock 310 is converted in a first section of the reactor. The feedstock 310 is then isomerized in the second section of the reactor 322 and xylenes are converted to the thermal equilibrium concentration. The effluent from the ethylbenzene conversion/xylene isomerization reactor 322 provides a first effluent stream 324 which is sent to a xylene splitter 312 that removes C₉⁺ aromatics (and in some embodiments of the invention *ortho*-xylene) from the first effluent stream 324 as by-products 313 and provides a first depleted stream 334. Separators 325 and 327 are used to remove benzene 326 and toluene 328 from the first depleted stream 334. The first depleted stream with benzene and toluene removed 330 is processed in a *para*-xylene recovery unit 318 where *para*-xylene is removed as a product 320. *Para*-xylene recovery is accomplished by selective sorption and/or crystallization. The effluent from the *para*-xylene recovery unit 318 provides a second depleted stream 336, which is fed to an isomerization unit 332 where xylenes are converted to the thermal equilibrium concentration and most of the ethylbenzene is converted benzene and ethane. The effluent from the isomerization unit 332 provides a second effluent stream that is recycled into the first depleted stream 329 downstream of the benzene removal stage 326 but prior to the removal of toluene 328. Toluene 328 removed from the first depleted stream 329 downstream of the benzene removal stage 326 is recycled into the feedstock 310 upstream of the ethylbenzene conversion/xylene isomerization reactor 322.

As in case of the first example, a first slip stream 340 of the second depleted stream 336 from the *para*-xylene recovery unit 318 is recycled into the feedstock 310 upstream of the ethylbenzene conversion/xylene isomerization reactor 322 and a second slip stream 342 may be recycled into the first effluent stream 324 of the ethylbenzene conversion/xylene isomerization reactor 322.

In a preferred embodiment, the toluene concentration of the feedstock 310 is increased by adding toluene to the feedstock 310 to change the composition. In a most preferred embodiment, toluene is added to the feedstock 310 so as to increase the ratio of toluene to xylenes. The toluene can come from an external source or it can be removed from the process downstream of the ethylbenzene conversion unit 322 and recycled into the feedstock 310. After the toluene concentration has been increased, the concentration of toluene in the feedstock 310 is from 1% to 25% by weight, with a preferred concentration of 10% to 20% by weight.

In another preferred embodiment, in addition to recycling the second depleted stream 336 into the feedstock 310 and optionally into the first effluent stream 324 through a slip stream 340 and 342, the toluene concentration of the feedstock 310 is increased by adding toluene to the feedstock 310. In this embodiment, the ratio by weight of the slip stream 340 to the toluene that is added is from 1 to 1 to 10 to 1, with a preferred ratio of from 3 to 1 to 5 to 1.

## Claims

1. A process for producing *para*-xylene from a C₈⁺ aromatic feedstock which contains xylenes and ethylbenzene, the process comprising the steps of:
(a) subjecting said feedstock to ethylbenzene conversion in a first stage to provide a first effluent stream;
(b) removing C₉⁺ aromatics and, optionally, *ortho*-xylene, from said first effluent stream to provide a first depleted stream;
(c) recovering *para*-xylene from said first depleted stream to provide a second depleted stream;
(d) isomerizing xylenes in said second depleted stream to provide a second effluent stream;
(e) recycling said second effluent stream into said first depleted stream prior to *para*-xylene recovery; and
(f) recycling a slip stream from said second depleted stream into said feedstock.

2. A process according to Claim 1, which further comprises the step of adding toluene to the feedstock prior to step (a).

3. A process according to Claim 2, wherein at least part of the toluene which is added to the feedstock is obtained by separation of toluene from said first depleted stream.

4. A process according to Claim 3, wherein said second effluent stream is recycled into said first depleted stream prior to said toluene separation.

5. A process according to any preceding Claim, which further comprises recycling a slip stream from said second depleted stream into said first effluent stream.

6. A process according to any preceding Claim, wherein up to 50% of said second depleted stream is recycled.

7. A process according to any preceding Claim, wherein step (a) also effects isomerization of the xylenes in the feedstock.

## Patentansprüche

1. Verfahren zur Herstellung von para-Xylol aus einem aromatischen C₈⁺-Einsatzmaterial, das Xylole und Ethylbenzol enthält, bei dem
(a) das Einsatzmaterial in einer ersten Stufe einer Ethylbenzolumwandlung unterworfen wird, um einen ersten Ausflussstrom zu liefern;
(b) C₉⁺-Aromaten und gegebenenfalls ortho-Xylol aus dem ersten Ausflussstrom entfernt werden, um einen ersten verarmten Strom zu liefern;
(c) para-Xylol aus dem ersten verarmten Strom gewonnen wird, um einen zweiten verarmten Strom zu liefern;
(d) Xylole in dem zweiten verarmten Strom isomerisiert werden, um einen zweiten Ausflussstrom zu liefern;
(e) der zweite Ausflussstrom vor der para-Xylol-Gewinnung in den ersten verarmten Strom zurückgeführt wird; und
(f) ein Nebenstrom aus dem zweiten verarmten Strom in das Einsatzmaterial zurückgeführt wird.

2. Verfahren nach Anspruch 1, bei dem dem Einsatzmaterial vor Stufe (a) Toluol zugegeben wird.

3. Verfahren nach Anspruch 2, bei dem mindestens ein Teil des Toluols, das dem Einsatzmaterial zugegeben wird, durch Abtrennen von Toluol aus dem ersten verarmten Strom erhalten wird.

4. Verfahren nach Anspruch 3, bei dem der zweite Ausflussstrom vor der Toluolabtrennung in den ersten verarmten Strom zurückgeführt wird.

5. Verfahren nach einem der vorhergehenden Ansprüche, bei dem ferner ein Nebenstrom aus dem zweiten verarmten Strom in den ersten Ausflussstrom zurückgeführt wird.

6. Verfahren nach einem der vorhergehenden Ansprüche, bei dem bis zu 50 % des zweiten verarmten Stroms zurückgeführt werden.

7. Verfahren nach einem der vorhergehenden Ansprüche, bei dem Stufe (a) auch eine Isomerisierung der Xylole in dem Einsatzmaterial bewirkt.

## Revendications

1. Procédé pour la production de para-xylène à partir d'une charge aromatique d'alimentation en C₈⁺ qui contient des xylènes et de l'éthylbenzène, procédé comprenant les étapes consistant :
(a) à soumettre ladite charge d'alimentation à une conversion d'éthylbenzène dans une première étape pour produire un premier courant effluent ;
(b) à séparer les composés aromatiques en C₉⁺ et, facultativement, l'orthoxylène dudit premier courant effluent pour produire un premier courant épuisé ;
(c) à récupérer le paraxylène à partir dudit premier courant épuisé pour produire un second courant épuisé ;
(d) à isomériser les xylènes dans ledit second courant épuisé pour produire un second courant effluent ;
(e) à recycler ledit second courant effluent dans ledit premier courant épuisé avant la récupération du paraxylène ; et
(f) à recycler un courant de dérive provenant dudit second courant épuisé dans ladite charge d'alimentation.

2. Procédé suivant la revendication 1, qui comprend en outre l'étape d'addition de toluène à la charge d'alimentation avant l'étape (a).

3. Procédé suivant la revendication 2, dans lequel au moins une partie du toluène qui est ajouté à la charge d'alimentation est obtenue par séparation du toluène dudit premier courant épuisé.

4. Procédé suivant la revendication 3, dans lequel ledit second courant effluent est recyclé dans ledit premier courant épuisé avant ladite séparation du toluène.

5. Procédé suivant l'une quelconque des revendications précédentes, qui comprend en outre le recyclage d'un courant de dérive provenant dudit second courant épuisé dans ledit premier courant effluent.

6. Procédé suivant l'une quelconque des revendications précédentes, dans lequel une proportion allant jusqu'à 50% dudit second courant épuisé est recyclée.

7. Procédé suivant l'une quelconque des revendications précédentes, dans lequel l'étape (a) effectue également l'isomérisation des xylènes dans la charge d'alimentation.
